# EUROPEAN PATENT APPLICATION

(11) **EP 1 334 729 A1**
(43) Date of publication of application: **13.08.2003**
(21) Application number: 02250834.5
(22) Date of filing: 07.02.2002
(51) Int. Cl.: A61K 38/45, C12N 9/10, A61P 37/00, A61P 27/00, A61P 21/00

(54) **Therapeutic use of non-neurotoxic clostridium botulinum toxin type C2**

(71) Applicant: Botulinum Toxin Research Associates, Inc., Quincy, Massachusetts 02169 (US)
(72) Inventor: Borodic, Gary E., Canton, Massachusetts 02021 (US)
(74) Representative: Gardner, Rebecca

(57) **Abstract**

The invention relates to clostridial toxin compositions and their use for the treatment of diseases, particularly diseases which are associated with pain, inflammation and irritation, as well as movement disorders. In particular, the invention provides a botulinum toxin which substantially lacks neurotoxic activity, or a biologically active component thereof which substantially lacks neurotoxic activity, for use in therapy.

## Description

This invention relates to clostridial toxin compositions and their use for the treatment of diseases, particularly diseases which are associated with pain, inflammation and irritation, as well as movement disorders. The invention offers an improvement on the prior art by eliminating the muscle-weakening side effect of prior-art botulinum toxin which act as chemodenervating agents. This is achieved by eliminating the neurotoxin component of the botulinum toxin.

Botulinum toxin exists as multiple immunotypes (A-G), which have been investigated as to specific medical properties. The immunotypes share little cross reactivity and have been shown to differ in chemical composition and biological behavior when injected at sublethal doses to produce a regional dose-dependent effect. Differences in biological activity between the various immunotypes include (1) differing durations of action at the injection site and (2) differing regional denervation potencies, measured as the relative quantity of LD 50 units required to produce a given regional or clinical effect.

Application of type A botulinum toxin to human essential headache disorders was originally identified in myofascial tension type headaches (see Acquardro and Borodic, Treatment of myofascial pain with botulinum A toxin. Anesthesiology. 1994 Mar;80(3):705-6), and later, as a coincident finding, for migraine headache. The coincident finding and application for migraine was noted as botulinum toxin was being used as a neurotoxin to remove forehead wrinkles. Regional facial muscular weakening effaces facial and forehead dynamic lines associated with aging. Such dynamic lines are produced by facial muscle tone as forces are transmitted from facial muscles to dermal attachments of these muscles in facial skin. The neurotoxin component was thought active in the treatment of headaches and pain during this period. For example, US Patent # 5,714,468 teaches that the neurotoxin (that component of botulinum toxin which causes neuromuscular paralysis) is the active agent and mechanism by which botulinum toxin is effective for the treatment of migraine and other forms of pain.

Migraine and tension headaches are a major cause of loss of productivity for those afflicted, usually due to pain and associated systemic symptoms. The syndrome of migraine and other essential headaches is characterized by severe throbbing headaches often made worse by physical activity and associated with aversion to light and sound. The syndrome often, but not always, includes nausea and/or sometimes vomiting as major components. The pain is often unilateral or localized to a portion of the head. The condition is episodic in nature, with episodes typically lasting 4-72 hours.

Tension headache is the most common type of essential headache and is characterized by head pain not associated with any structural lesions, often not associated with nausea, and occurring more frequently and less episodically than migraine headache.

Botulinum toxin has been effectively used for a number of medical conditions involving muscular disorders such as neurologic blepharospasm, hemifacial spasm, jaw dystonia, temporal mandibular joint disease, headaches syndromes, hand dystonia, spasticity after cerebrovascular accident and with cerebral palsy and multiple sclerosis, spasmodic torticollus, achalasia, and bruxism. Additionally, certain primary pain disorders have been treated including post surgical head and neck pain after brain surgery, certain forms of low back pain, tension headaches, and temporal mandibular joint syndrome.

Efficacy for both myofascial pain and migraine headaches has been shown in double-blind placebo-controlled trials. However, the mechanism of action has not been clearly explained, as botulinum toxin has been thought to exert its beneficial effects for most indications by causing decreased muscle tone and contractility. The limiting factor inherent in using botulinum toxin for the treatment of pain is the attendant weakness produced by the effects of botulinum toxin on the neuromuscular junction, causing impairment in neuromuscular transmission, and subsequent muscular fiber atrophy which lasts for a period of 3-4 months. The neuromuscular effect results in weakness in active contractility and decrease in resting tone both in striated and smooth muscle. Such effects have consequences in the clinical setting which limit the application of botulinum toxin based pharmaceuticals application for certain forms of disease states in which muscle weakness would be undesirable and associated with complications. For instance, ptosis (drooping of the upper eyelid) is the major side effect caused by the injection of neurotoxin for the treatment of human headache disorders. This results from the neuromuscular paralytic effects of the neurotoxin component of the botulinum toxin molecule. Other deleterious effects of botulinum toxin injections associated with neuromuscular diseases include dysphagia associated with botulinum protein injection for adult onset spasmodic torticollis, ptosis and diplopia associated with the treatment of blepharospasm, hemifacial spasm, and vertical ocular deviation via injections of horizontal rectus muscles (extra-ocular muscles).

Until the present invention was made, it was thought that the use of botulinum toxin for the treatment of pain or inflammatory disease was not feasible, due to its weakening effect on muscle tissue caused by blocking acetylcholine release by its neurotoxin component. Since the neurotoxin component was thought to be responsible for the therapeutic effects of botulinum toxin, no solution to this problem was apparent. However, the invention described herein is based upon a compositional change to the botulinum exotoxin proteins used in medical practice that substantially eliminates neurotoxin activity, while maintaining a biological activity that is capable of suppressing pain and inflammation. Such compositional improvement allows the exotoxin protein to be used without the attendant risk of inducing muscle atrophy and weakness, while retaining a biological effect useful for the clinical relief of pain and discomfort.

It has now surprisingly been found that a clostridial toxin substantially lacking neurotoxin properties can be used in the therapeutic treatment of diseases, particularly those diseases which are associated with pain, inflammation and irritation, and movement disorders.

Thus viewed from one aspect, the invention provides a clostridial toxin which substantially lacks neurotoxic activity, or a biologically active component thereof, for use in therapy.

Viewed from a different aspect, the invention provides a pharmaceutical composition comprising a clostridial toxin which substantially lacks neurotoxic activity or a biologically active component thereof which substantially lacks neurotoxic activity and a pharmaceutically acceptable carrier or diluent.

A 'clostridial toxin' is one derived from the genus *Clostridium* and includes, *inter alia, Clostridium perfringens, Clostridium tetani, Clostridium spiroforme* and *Clostridium botulinum.*

Viewed from yet another aspect, the invention provides the use of a clostridial toxin which substantially lacks neurotoxic activity, or a biologically active component thereof which substantially lacks neurotoxic activity for the manufacture of a medicament for the treatment of pain, inflammation and irritation, diseases and disorders which are associated with pain, inflammation and irritation, and movement disorders.

The toxin is preferably a botulinum toxin and the invention will be described hereinafter with reference to the botulinum toxin, although it will be understood that toxins from other clostridial species may be employed.

The present inventor has shown botulinum toxin to have anti-inflammatory components, both in vivo in man and in animal experiments. This property is believed to explain the mechanism of action by which botulinum toxin exerts its beneficial effects in essential headache disorders (migraine and tension headaches) as well as in other medical conditions. Evidence that botulinum toxin acts on the inflammatory response associated with essential headaches can be categorized into clinical observations and animal experiments as disclosed herein.

The botulinum toxin of the present invention has many advantages over existing therapy for the treatment of essential headache disorders. These include (1) lack of systemic side effects, particularly compared to the krypton class of drugs, (2) long duration of action (3-5 months), (3) maintenance free therapy (no pills, no autoinjections), (4) high degree of efficacy.

The botulinum toxin or component thereof disclosed herein substantially lacks neurotoxic activity. A substance which lacks neurotoxic activity is not capable of producing death by action on a portion of the central or peripheral nervous system in such a manner as to destroy or critically impair organism function. In practical terms, the toxin in accordance with the invention is advantageous for therapy because it does not cause decreased contractility of striated muscles, does not block neuromuscular transmission and produce standard chemodenervation, and does not cause weakening around the injection site, these being properties associated with neurotoxic activity.

The botulinum toxin in accordance with the invention is a biologically active toxin and is therefore poisonous and capable of causing death. For example, the Swiss Webster mouse bioassay for lethality described in Example 6 herein is suitable for determination of biological activity. Any biologically active form of botulinum toxin or a biologically active component thereof is suitable, the only requirement being that it substantially lacks neurotoxic activity as defined above and hence a botulinum toxin obtained from any of the *Clostridium botulinum* strains may be used, provided that it is treated if necessary to substantially remove any neurotoxic properties. Thus in a preferred embodiment the botulinum toxin of the invention is prepared by biochemical separation to remove substantially all neurotoxin properties e.g. to remove type C1 from a preparation containing both types C1 and C2. Alternatively the toxin may be recombinantly produced by methods well known in the art.

Of the immunotypes of botulinum toxin, a mutant and unusual derivative subtype of botulinum toxin is botulinum toxin C2, which has been shown to have no neurotoxin properties. The proteinaceous materials derived from botulinum C2 strains are, however, biologically active and have been demonstrated to cause lethal effects by mechanisms other than neuromuscular paralysis.

Preferably, the botulinum toxin is a cytotoxin such as the type C2 botulinum toxin, which may be obtained for example from the *Clostridium botulinum* strains which produce it e.g. strains C and D (botulinum C2 toxin and its components can be prepared as described for example in Ohishi et al 1980 "Purification and characterization of two components of botulinum C2 toxin", Infect. Immun. 30; 668-673.) This protein is characterized by those skilled in botulinum toxin technology as a cytotoxin, which causes intestinal inflammation as a cause of toxicity, without inducing muscular weakness.

Whilst not wishing to be bound by theoretical considerations, it is thought that the C2 producing strain may have different genetic material from some other strains, causing the C2 toxin to lack the binding epitope to the presynaptic membrane at the neuromuscular junction. It is therefore understood that other strains of *Clostridium botulinum* or other bacteria, e.g. E. *coli* would be capable of producing botulinum toxins which substantially lack neurotoxic activity, for example by making appropriate genetic modifications to the toxin-producing organism. Thus in a preferred embodiment the toxin of the invention is purified from a bacterial strain expressing at least one cloned gene encoding a protein component of a non-neurotoxic clostridial toxin (preferably botulinum type C2). Genes encoding components I and II of botulinum toxin C2 have been cloned from *Clostridium botulinum* type C strain (C)-203U28 as disclosed in Fujii N. et al Biochem Biophys Res Commun 1996 Mar 18;220(2):353-9 and Kimura K. et al, Vet Microbiol 1998 Apr 30;62(1):27-34.

Thus, suitable strains from which the toxin may be obtained include: a naturally occurring strain making a non-neurotoxic cytotoxin, a naturally occurring strain making a C2 toxin, a genetically manipulated strain making non-neurotoxic cytotoxin, a genetically manipulated strain making C2 toxin, or a recombinant strain making non-neurotoxic cytotoxin or C2 toxin.

It is also preferred that the botulinum toxin in accordance with the invention be substantially free of other botulinum toxins which do have neurotoxic properties, such as the C1-type botulinum toxin. *Clostridium botulinum* C2 toxin, *Clostridium perfringens* iota toxin, and *Clostridium spiroforme* toxin act on ADP-ribosylate actin. Toxin-induced ADP-ribosylation disturbs the cellular equilibrium between monomeric and polymeric actin and traps monomeric actin in its unpolymerized form, thereby depolymerizing actin filaments and destroying the intracellular microfilament network (intracellular actin cytoskeleton). Furthermore, the toxins ADP-ribosylate gelsolin actin complexes. These cytoskeletal modifications may contribute to the cytopathic action of these toxins.

Suitable biologically active components of the botulinum toxin of the invention would include for example either of the two protein components I and II of type C2 botulinum toxin which have relative molecular masses of approximately 55 and 100 kDa, respectively, and are described for example in Ohishi et al., 1980, Infect. Immun. 30:668-673.

It will be appreciated by those skilled in the art that in practice, it may not be necessary to remove all neurotoxic properties from the toxin in order to achieve a clinically useful product which is an improvement over prior art botulinum toxin compositions. In other words, so long as the muscle weakening side effects of the prior art compositions are substantially reduced so as to be tolerable and preferably negligible, elimination of all neurotoxic activity, though preferred, is not necessary to obtain a clinically useful product. The term 'which substantially lacks neurotoxic activity' should be interpreted with these practical considerations in mind.

The muscular weakening effects of prior art forms of botulinum toxin which do not substantially lack neurotoxic activity are well documented and easily assessed. These prior art botulinum toxins are known to cause a form of reversible denervation atrophy, which is reversible in 3-4 months. The process of pre-axonal terminal sprouting with spread of acetylcholine receptors and cholinesterase has been well characterised by Duchenne and subsequently many others, and the histologic effects of treatment with prior art botulinum toxins are therefore well understood by persons skilled in the art. In most, if not all applications, weakness of injected muscles is easily assessed, e.g. with treatment of limb dystonia, improvement is not generally seem without detectable weakness. Hence the skilled person is readily able to distinguish prior art botulinum toxins from the botulinum toxin (or biologically active component thereof) of the present invention which substantially lacks neurotoxic activity.

The botulinum toxin of the invention is useful for the treatment of a variety of diseases and disorders. In particular, it is believed to be useful in the treatment of diseases and disorders associated with pain, irritation and/or inflammation and of these conditions *per se.* For example, essential headache disorders, photophobia, myofascial pain, regional muscle pain, dystonia (e.g. regional dystonia), head and neck pain (e.g. post-surgical/post-operative pain), back (especially lower back) pain, trigeminal neuralgia (typical and atypical) and temporal mandibular joint syndrome are all problems associated with pain and can be treated in accordance with the invention. Essential headache disorders include migraine and tension headaches.

Another preferred aspect of the invention is the treatment of inflammatory diseases and disorders, in particular for treating inflammation, edema, redness, itching and irritation associated with them. Such diseases and disorders include allergies such as allergic conjunctivitis and ocular inflammation, allergic blepharosconjunctivitis, asthma, eczema, hayfever, excessive salivation, tearing, otitis, anal fissures, cystitis, irritation, erythema and cholinergic urticaria. Treatment of the gastric or intestinal mucosa (e.g. gastrointestinal ulceration or gastric, small and large intestinal inflammation) is also encompassed, as well as treatment of rheumatoid arthritis which may afflict ligaments and/or joints, and vasculitis.

In addition to the treatment of pain and inflammatory disorders, the botulinum toxin of the invention is also thought to have utility in the treatment of movement disorders. Clearly, since the prior art botulinum toxin compositions were neurotoxins, the mechanism of action of a composition substantially lacking neurotoxic activity is different to that elucidated in the prior art. Thus in another preferred aspect the invention provides botulinum toxin which substantially lacks neurotoxic activity or a biologically active component thereof for use in the treatment of movement disorders.

Such disorders include, but are not limited to neurologic blepharospasm, hemifacial spasm, spasticity (e.g. after cerebrovascular accident), cerebral palsy, multiple sclerosis, spasmodic torticollis, achalasia and bruxism.

Other disorders which may be treated in accordance with the invention include facial pain syndrome, trigeminal neuralgia, chronic incisional pain syndromes, back pain, compressive pain syndromes, myofascial pain, essential headache, blepharitis, skin inflammation, joint inflammation and muscle inflammation.

In all applications of therapeutic treatment, properties associated with the non-neurotoxic nature of the botulinum toxin of the invention allow an improvement over prior art treatments. Thus for example a patient may be treated in accordance with the present invention without inducing muscular weakness or atrophy or other deleterious weakness associated with these conditions.

The region of the body of the patient to be treated may be at any affected site, but in particular includes the head and neck.

In general, the botulinum toxin of the invention may be administered for therapeutic purposes in any suitable form. Preferably it may be administered in such a way as to allow it to be applied to a defined region or part of the body, and hence topical methods of application or injection are preferred. For example, the botulinum toxin is preferably applied directly to the body site to be treated. Intramuscular or intravenous administration may be suitable. For example, in the treatment of head pain, injection of the botulinum toxin into the superficial soft tissue of the head is preferred. Oral administration is also suitable, and in the case of gastrointestinal disorders and other internal inflammation, it is preferred.

Dosage of the botulinum toxin of the invention may be adapted for the particular treatment regimen which is required. Thus, the skilled person may determine an acceptable level of dilution by methods well known in the art in order to provide the desired therapeutic effect without producing adverse toxic reaction in the subject. In general, however, dosage is conveniently quantified by the LD50 Swiss Webster mouse bioassay and is preferably in the range of from about 0.1 to about 50,000. One LD50 unit is equal to that quantity of botulinum toxin causing a lethal effect in 50% of a population of 20-30 g white mice as described in the standard Schantz-Kauter bioassay using Swiss Webster mice. Depending on various factors, such as the size and position of the affected area, the dosage range may vary. For example, for small affected areas, such as pretarsal orbicularis, 0.1-500 LD50 units may be preferable; for large areas of neck and back, 20,000-30,000 LD50 units may be preferable. The most preferred dosage range is either from about 5 to about 20 LD50 units or from about 20 to about 40 LD50 units or from about 20 to about 2000 LD50 units.

C2 toxin is useful in frozen, freeze-dried, or liquid form.

Preferably, the botulinum toxin is prepared and stored in the form of a liquid or freeze dried composition, and it may be diluted or otherwise treated if necessary prior to administration. However, where components of the C2 botulinum toxin, particularly component II is concerned, liquid formulations are preferred. In the pharmaceutical composition of the invention, preferred adjuvants include those which serve to stabilise the botulinum toxin, such as Clostridium derived proteins. Stabilizing proteins could be hemagglutinin, or non-neurotoxin, non-hemagglutinin. Human serum albumin may be useful for stabilization as well.

Certain embodiments of the invention will now be described by way of example only in the following Examples and with reference to the drawings in which:
Figure 1 shows the duration of protective effect over 6 months of botulinum toxin type C2 for a series of 6 animals sensitised and exposed to short ragweed pollen spores.
Figure 2 is a graph showing the rub time in sensitized guinea pig injected with peribulbar botulinum toxin and subsequently exposed to allergen (Ragweed).

### TECHNICAL DISCUSSION AND EXAMPLES

Type A botulinum toxin has been used for the past 16 years to treat various forms of facial movement disorders, including Blepharospasm, hemifacial spasm, bruxism, and synkinetic facial movements after chronic facial palsy. This substance has also proven substantial utility for the treatment of spasmodic torticollis, spasticity associated with cerebral palsy, stroke, occupational hand cramping, and speech disorders (spasmodic dysphonia). In each of these applications, the mechanism of action had been postulated to involve weakening of a fixed volume of muscle around the area injected for a period of 10-18 weeks, with complete reversal of the weakening effect after that time. During the period after the injection, the weakening is correlated to (1) blockage of release of acetylcholine from the presynaptic nerve terminal at the neuromuscular junction, (2) atrophy similar to motor nerve denervation atrophy in the area over which the toxin diffuses, (3) decreased contractility within the muscles over which the toxin diffuses, (4) motor nerve terminal sprouting from the motor axon terminal, (5) spread of acetylcholinesterase and acetylcholine receptors from the post synaptic membrane, and (6) reversibility of the above findings within the denervation field after 10-18 weeks.

Collectively the above describes a cycle that has been well characterized in the observations of Duchenne (Scott AB Botulinum toxin injection of eye muscles to correct strabismus. Trans Am Ophthalmol Soc 1981;79:734-70). Additionally, it has been well established that botulinum toxin has local effects on autonomic nerve ganglion and nerve function. (MacKenzie I, Burnstock G, Dolly JO The effects of purified botulinum neurotoxin type A on cholinergic, adrenergic and non-adrenergic, atropine-resistant autonomic neuromuscular transmission.Neuroscience 1982 Apr;7(4):997-1006).

The medical utility of botulinum toxin has been based primarily on the neuromuscular effects of botulinum neurotoxin, as the neurotoxin generates the cycle described in steps (1)-(6) above. The definition of a neurotoxin is an agent capable of producing death by action on a portion of the central or peripheral nervous system in such a manner as to destroy or critically impair organism function. In the case of botulinum neurotoxin, the action is at the level of the neuromuscular junction, leading to disseminated weakness with paralysis of critical muscles such as the muscles driving respiratory ventilation. The lethal effect, which occurs at a critical point of muscular weakness, is asphyxiation and suffocation. The pharmacological principle governing the utility of botulinum toxin in the treatment of human diseases is that a regional effect occurs at a diluted concentration-dose remote from the lethal concentration-dose. Stated another way, this principle is the property of neurotoxin that allows a regional effect at a neurotoxin dilution and concentration substantially lower than that concentration that would cause a lethal systemic effect for the various types of botulinum neurotoxin used. That lower concentration allows for regional muscular weakening, which has been thought to be the sole mechanism by which the neurotoxin exerts its beneficial action in diseases involving spastic or involuntary movement.

Despite this scientific understanding of botulinum toxin as a neurotoxin, prior to the present invention there was insufficient understanding of the biological tissue effects to explain the observed utility for other medical conditions such as the treatment of human pain such as occurs in essential headache disorders, myofascial pain, and certain pain components associated with dystonias. Also, there was previously no explanation of the mechanism by which botulinum toxin is effective in reducing inflammation within the denervation field. Indeed, the invention is based in part on the recognition that the action of botulinum toxin as a neurotoxin, a substance acting at the neuromuscular junction causing muscular weakness, fails to provide a sufficient basis for the mechanism by which utility is achieved for conditions which are not associated with abnormality in movements.

Described in the following examples is the bioeffect thought critical to the property of botulinum toxin that is directly or indirectly related to its ability to relieve human pain. Also described are methods by which this property can be chemically dissociated from the neurotoxin component (muscle-weakening component) of the botulinum toxin pharmaceutical agent, thereby generating a new perfected botulinum-derived pharmaceutical agent capable of eliminating the undesirable muscle weakness associated with injection of prior-art botulinum toxin preparations into a diseased area.

The following Examples used C2 toxin from the well known *Clostridium botulinum* C strain 'Stockholm', as described, for example, in J. Protein Chem. 1999 Nov; 18(8): 885-92. Botulinum type C stock is known which produces neurotoxic type C1 and non-neurotoxic type C2 and other stock produces just C2, the purification protocol described herein is appropriate for both stock types.

### EXAMPLE 1

Botulinum toxin has been found by the inventor to have the ability to relieve pain and irritation or both *in vivo* in man and in animal experimentation in certain peri-ocular diseases in patients not suffering from ocular movement diseases such as essential blepharospasm or Meige syndrome. Case Histories:

### Case 1:

This 71 year old woman suffered from Glaucoma causing severe visual loss. She had been maintained on a medicinal regimen consisting of carbonic hydrase inhibitors, pilocarpine, Alphagan-TM, Xalatan 0.005% to maintain adequate ocular pressure control. Additionally, argon laser photocoagulation to the trabecular meshwork in each eye was given to maximal degree. She developed conjunctival redness, pain and irritation on the surface of both eyes thought to be secondary to medical allergy or side effect. Because of the need to treat the glaucoma and the patients unwillingness to undergo surgical procedures, injections of commercially available botulinum type A toxin were administered into the peri-ocular tissues in the usual locations to suppress irritation and pain associated with anti-glaucoma medication use. The botulinum toxin suppressed pain, irritation and allowed this patient continued medicinal treatment for glaucoma.

### Case 2:

This 42 year old woman suffered for many years from ocular irritation associated with seasonal allergic conjunctivitis as well as peri-ocular pain and discomfort. She had a past history of severe hayfever and allergic type cataract. Despite the use of conventional medication for the treatment of allergy and ocular inflammation, her symptoms continued. Botulinum toxin was administered to the peri-ocular region for the treatment of ocular irritation associated with allergic conjunctivitis with excellent sustained results over a period of 3-4 months, consistent with the known duration of action of botulinum toxin.

### Case 3:

This 75 year old man suffered from chronic severe ocular irritation and discharge from chronic blepharitis not responsive to cortico-steroid type drugs, mast cell stabilizers, sympathomimetic agents as well as anti-histamine agents. Botulinum toxin was injected into the peri-ocular areas in the usual injection location to treat movement diseases of the eyelids. Decreased discharge, irritation discomfort, rubbing and redness ensued.

This unique property has not been previously appreciated or defined and in fact may explain the mechanism of action which botulinum toxin exerts its beneficial effects in essential headache disorders, such as migraine and tension headaches as well as benefiting other medical conditions. Evidence that botulinum toxin acts on the pain generating nerves with essential headaches can be categorized into clinical observations and animal experimentation as disclosed in the remaining Examples.

### EXAMPLE 2

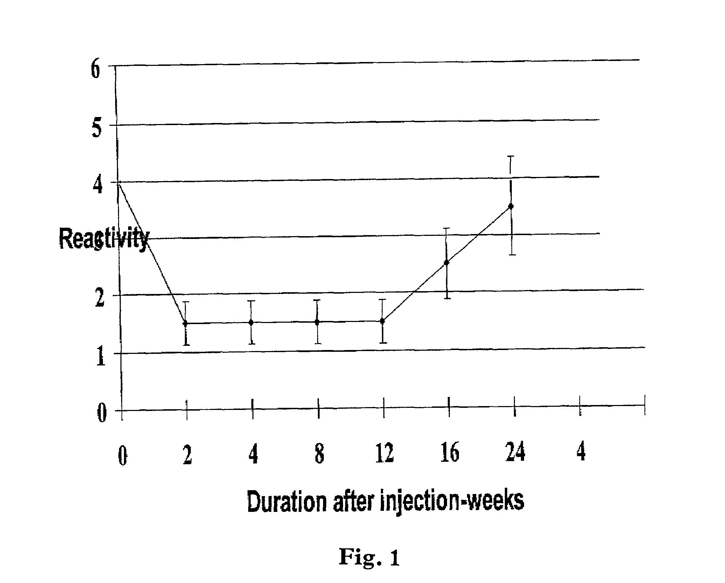

Efforts to explain the critical property of botulinum toxin capable of causing an improvement in pain associated with dystonia, myofascial pain, essential headaches, photophobia associated with essential blepharospasm, pain associated with temporal mandibular joint syndrome as well as other syndromes initially came from observations seen in Figure 1.

This 53 year old woman and 44 year woman experienced usual flushing face and disseminated itching following physical exertion. The face demonstrated hives, associated with the flushing.

The 53 year old woman's past medical history was significant Bell's palsy for which she received a botulinum type A injection for the treatment of forehead asymmetry. She noted that after the botulinum toxin was injected into the forehead, there would be white blotches appearing on the forehead in which there was no flushing, and no hive formation, blocked urticaria within the denervation field as can be seen in Figure 1. This patient noted this effect after physical exertion consistently for a period of three months after the botulinum type A injections and the effect slowly faded thereafter. This duration of effect is typical for botulinum type A injections.

The 44 year old woman received the botulinum toxin for treatment of hyperkinetic forehead lines. She note incidental improvement both in her essential headaches as well as marked depression in facial flushing, hive formation, and itching within the effective geometric field in which the given dose is noted to diffuse.

Both these patients exhibited the syndrome of cholinergic urticaria.

White blotches were seen on the forehead of a patient who has been injected with botulinum toxin type A. The blotches indicate blocked urticaria formation within the diffusion (denervation) field of botulinum toxin. The patient suffers from typical cholinergic urticaria. The blocked area of urticaria, which includes edema, erythema, and altered sensation corresponds to the usual diffusion field of botulinum toxin at doses between 5-20 LD 50 units.

A pattern of erythema was seen on the neck of a second patient with dystonia and pain, who was injected with botulinum type A toxin. Note that in this patient both the erythema and the pain was improved within the denervation field surrounding the injection (the diffusion field of the botulinum toxin).

A pattern of erythema was seen on the neck of a third patient with cervical dystonia who was injected with type A botulinum toxin. A block in the erythema surrounding the injection site was seen. This patient has been well documented to exhibit circulating neutralizing antibodies to the neuromuscular paralyzing effects of the immunotype A *Clostridium botulinum* toxin. No muscular atrophy is seen in injected muscle yet there is a block in the erythema surrounding the injection site in a dimension equivalent to the diffusion potential for 20-40 LD 50 units of botulinum toxin. As neutralizing antibodies to the neuromuscular effect of botulinum did not block this anti-pain and anti-inflammatory effect, the anti-inflammatory effect may be the result of a different immuno-reactive epitope on the botulinum toxin proteins.

The syndrome of cholinergic urticaria is typically associated with urticarial eruption after exertion; sometimes the condition is also associated with symptoms of asthma. The pathophysiology has been linked with increased release of circulating histamine and mast cell degranulation. Histamine is a well-known modulator of sensation, inducing itching or frank pain in patients known to be predisposed to the development of essential headaches. As the above bioeffect appeared novel and not well explained by existing understanding of botulinum efficacy, efforts were made to confirm the effect on human mast cells in an experimental *in vivo* laboratory experiment. As the above-noted bioeffect appeared novel and not well explained by existing understanding of botulinum toxin efficacy, efforts were made to confirm the effect on human mast cells in an in vivo laboratory experiment.

Botulinum toxin is well known to be effective in cervical dystonia. The relief of pain had been thought in the past to be caused by the relaxation of tight muscles caused by the neurotoxin properties of the agent. In the case of the second patient described above who presented with severe pain and erythema to the neck, botulinum toxin was given in the mid position of the neck, over the sternomastoid muscle. Two weeks after injection both the pain and the erythema was diminished over the area injected to the exact extent area expected for the dose of botulinum toxin to diffuse from the injection site.

The third patient, who suffered from dystonia and pain, developed neutralizing antibodies to the neurotoxin property of the injected botulinum toxin formulation, and subsequently failed to demonstrate weakness and atrophy of muscular tissue after injection. Using red sensitive photography however, the injected botulinum toxin still both relieved pain and depressed signs of neck erythema.

Both cases indicate the neurotoxin-weakening property does not fully explain the pain relieving properties of botulinum toxin. The pain relieving property should be considered as a separate biologic effect of the material. The invention described herein represents a method of isolating such effects from the neuromuscular weakening effects by the development of an altered botulinum toxin composition.

### EXAMPLE 3

This example is a demonstration of botulinum type C2 as a specific inhibitor of inflammation in a sensitized animal model, and reduction to practice utilizing botulinum C2 as a (1) therapy for inflammation (more specifically, therapy for irritation and discomfort associated with allergic conjunctivitis) and (2) therapy for migraine and tension headache treatment (more specifically therapy for pain and irritation associated with various forms of headaches, periocular and facial pains).

A Hart Bartley guinea pig (a guinea pig prone to type 1 hypersensitivity reactions) was sensitized to pollen spores (short ragweed pollen- Ambrosia artemisfolia), with aerosolized spores sprayed into the conjunctiva of the animals for a period of two weeks. Prior to this exposure, the animals had no reaction to the pollen, with the conjunctival membranes appearing white and quiet after exposure. After two weeks however, animals were again exposed to the short ragweed pollen, which caused acute edema, erythema, itching, flame haemorrhages within the conjunctiva, and distortion of the eyelids. This animal model has been pathologically characterized as being associated with measurable mast cell degranulation histologically, when pollen spores were exposed to sensitized conjunctiva.

The typical reaction was seen 15 minutes after short ragweed pollen spores were exposed to the sensitized animal. Red eye, edema and distortion of the lower eyelid position was seen in the animal, showing the effect of exposure of the conjunctiva to short ragweed pollen spores on an animal sensitised to the spores. Protection by botulinum toxin was also seen from the inflammatory response after exposure to the short ragweed pollen. Botulinum toxins type A, type C, and type C2, when injected, protect against the inflammatory effects associated with hypersensitivity to short ragweed pollen and duration of reflex scratching movements, hence demonstrating the anti-inflammatory, anti-irritative and suppressant effects of botulinum toxin on reflex movements and in the animals' orbit tissues. Rubbing the inflamed irritated eye is quantified over a period of 15 minutes after exposure to the pollen. The sensitized Guinea pig conjunctiva is injected with botulinum C2 preparation. The irritation quantified by rubbing behavior is diminished significantly (P<0.05, n=6).

The duration of the protective effect (anti-inflammatory) is demonstrated in Figure 1 for a series of 6 animals followed for 6 months. Measurement of involuntary reflex scratching movements in each animal were compared on the side given the C2 preparation in the periorbital region vs the side given controls saline injections within the same region. Reflex scratching movements were quantified according to duration of this movement over a 15 minutes period after an exposure to the allergen. Comparison were made using the Wilcoxon statistic.

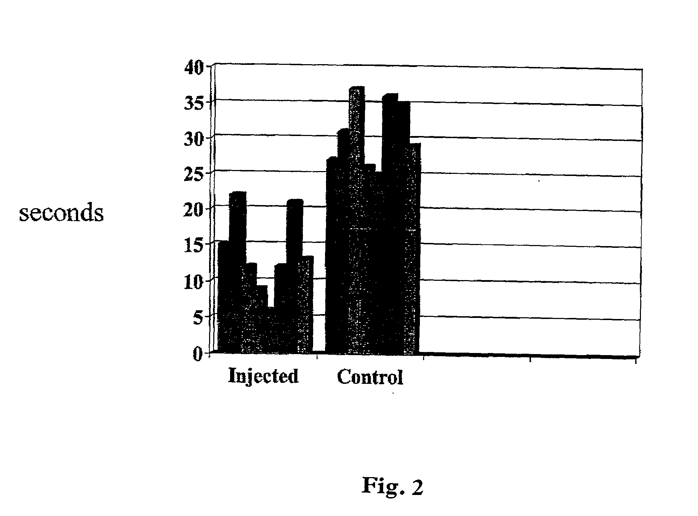

The behavioural reaction to ragweed pollen in sensitised guinea pig conjunctiva was characterised by rubbing of the irritated eye. Rubbing the inflamed irritated eye was quantified over a period of 15 minutes after exposure to the pollen. We found the rubbing behaviour in response to ragweed pollen in sensitised guinea pig conjunctiva was diminished due to injections of botulinum C2 (see Figure 2).

The sensitized Guinea pig conjunctiva is injected with botulinum C2 preparation. The irritation quantified by rubbing behavior is diminished significantly (P<0.05, n=6).

Given the demonstrated efficacy in cholinergic urticaria (Example 2) and demonstrated anti-irritating, analgesic effect in the allergic animal models (Example 3) measuring immediate hypersensitivity reactions, such effects are thought to represent a possible impairment in mast cell degranulation phenomenon. The hypersensitivity model using the Hart Bartley guinea pig has been well characterized histologically as representing a mast cell degranulation model. It appears that C2 botulinum toxin either directly or indirectly is influencing the system which involves mast cells, histamine, possibly serotonin, and other related autocoids in such a fashion to cause a blocked physiologic response important to the pathogenesis of certain forms of pain mechanisms both with respect to pain with dystonia and primary pain syndromes. Furthermore the depression of involuntary scratching type movements to anti-histamine type biologic effect created by the C2 preparation. The C2 effectively behaves as a movement suppressant (reflex scratching) without causing weakness.

Due to release of autocoids, such as various forms of prostaglandins and leukotrienes as well as other formed and generated local mediators, and as an obvious clinical observation, it is expected that the inflammatory response will be associated with pain degeneration by mechanisms relating to alterations of mast cell secretion or degranulation.

### EXAMPLE 4

In a known physiological assay, the relationship between mast cell degranulation and pain is clearly demonstrated. After a type 1 hypersensitivity response is demonstrated on the forearm of a person with known allergy to an introduced allergen, there appears to be a typical wheal and flare response associated with the sensory perception of itching. This is known as the immediate response. After a period of 6-8 hours, a late response is occasionally noted, characterized not by itching but rather tenderness and pain. The immediate response is thought to be associated and effected by preformed mediators such as histamine, whereas the late response is thought to be associated with the leukotrienes and prostaglandins. The prostaglandins and leukotrienes are important in the late phase reaction and are associated with pain generation. Compounds known to block prostaglandin derivatives such as indomethacin and corticosteroids will also block the late phase reactions associated with mast cell degranulation. In cellular systems, dependent on the adhesion of mast cells, there has been observed an increase or decrease in secretion induced by C. botulinum C2 toxin. In suspended mast cells, pretreatment with botulinum C2 toxin causes inhibition of secretion. In contrast, in adherent mast cells, the destruction of the cytoskeleton by botulinum C2 toxin causes increase in secretion. Thus, the signaling is largely effected by adhesion of mast cells in cellular in vitro studies, and mast cells have the capability of being influenced by the non-neurotoxin botulinum C2.

### EXAMPLE 5

### PREPARATION OF BOTULINUM TOXIN SUBSTANTIALLY LACKING NEUROTOXIC ACTIVITY

A botulinum toxin suitable for the present invention may be prepared as follows. A preparation is made consisting of a *Clostridium botulinum* strain which produces C2 cytotoxin. Toxin production is accomplished with appropriate agents to procure the maximum number of LD 50 units per ml of culture solution. LD 50 units are determined using the mouse bioassay, and the preparation may either be freeze-dried for the purpose of preservation and stability or stored in dissolved form. A known quantity of bioactivity as determined by LD 50 is injected into the area in which pain, headache, or inflammation have been diagnosed by the clinician. A quantity for injection is chosen by the clinician based on LD 50 units.

Botulinum C2 is characterized as a non-neurotoxin, capable of causing increased vascular permeability, fluid accumulation in ligated intestine, and rounding of tissue cultured cells. Bioassay for the activity can be accomplished by a "time to mouse death method" after intravenous infusion, or by intrperitoneal injection. Anti-neurotoxin sera to botulinum toxin may be used to confirm complete neutralization of the neurotoxin prior to bioassay. Also, anti-C2 antisera may be used to characterize activity of C2 preparations. Preparation may be accomplished by biochemical isolation from spent cultures, or by recombinant production using either *E coli*, a Clostridium expression system, or another heterologous expression system, given that genes encoding the C2 protein have been elucidated. (See Fujii N. et al Biochem Biophys Res Commun 1996 Mar 18;220(2):353-9 and Kimura K. et al, Vet Microbiol 1998 Apr 30;62(1):27-34).

Botulinum C2 cytotoxin has been shown to consist of two protein components not covalently bound, segment I and segment II, both required for the biological activity of the toxin. The following represents examples of preparations that can be used to isolate the C2 property from neurotoxin properties and/or other constituents of botulinum toxin. Because the biological activity of component II may be affected by freeze-drying, a liquid formulation of the material would be preferred.

Following is a detailed description of an example method for isolating botulinum toxin C2.

Inoculum medium. Prepare 250 mls/carboy of media containing 2% trypticase peptone, 1% proteose peptone, 1% yeast extract, 1.0% glucose and 0.1% cysteine HCl, ph 7.4.

Inoculation with type C stock. Inoculate each flask with 0.5 ml of thawed working stock culture of C botulinum type C. Incubate for 24 hours.

Production medium. Prepared 9.5 L carboy of media containing 2% trypticase peptone, 1% protease peptone, 1% yeast extract, 1.0% glucose (prepared and autoclaved separately in 400 ml of distilled water) and 0.1% cysteine HCl, pH 7.4.

Inoculate production medium. Add glucose solution and 250 ml of inoculum to each 9.5 L carboy. Incubate at 30 degrees C for 6 days.

Harvest toxin. Toxin was harvested by addition of 2g/L RNA and dropping the pH to 3.4 by addition of 3N sulfuric acid. Toxin is allowed to settle for at least 16 hours and then collected by centrifugation.

Wash toxin. Toxin is washed with 1 liter of distilled water and allowed to settle. Washed toxin is collected by centrifugation (10K rpm for 10 min at 10 degrees C) and the toxin is extracted from the pellets.

Na phosphate extraction of the toxin. Thoroughly suspend the toxin pellets in 600 ml of 0.2 M Na Phosphate buffer + 0.5 M NaCl, pH 7.0, and extract at room temperature for 2 hours. The extract is centrifuged and the new extraction repeated on pelleted material with an additional 400 ml of the same buffer for 2 hours.

Ammonium sulfate precipitation. Precipitation of the extract by the addition of ammonium sulfate (39g/100ml).

The above steps may be repeated if necessary or desired to produce a purer preparation.

Prepare toxin for chromatography. Collect the precipitate by centrifugation at 8000 rpm for 10 min. Dissolve the precipitate in 50 nM of sodium citrate, pH 5.5, using as small volume as possible.

Dialysis of toxin solution. Toxin solution is dialyzed against 3X changes in citrate buffer overnight at 4 degree C and centrifugation ensues.

DEAE-Sephadex A-50 chromatography. Centrifuged material is chromatographed at room temperature on a -1 DEAE sephadex A-50 column (5cm X 50 cm , Sigma Chemical Co., St Louis , MO) equilibrated with the same buffer. One tenth of the column volume or less is chromatographed in a single passage with the toxin complex eluting in the first column without a gradient. Fractions from the protein peak which have a 260/278 absorbance ratio of less than 0.70 are pooled. The material must have a specific toxicity of > 10 (6) LD 50/mg. Pooled toxin is precipitated with 60% ammonium sulfate.

Separation of Component I and II may be accomplished with further chromatographic separation to achieve further purification.

The Swiss Webster mouse bioassay for lethality of the purified preparation is utilized to quantify bioactivity. Anti-neurotoxin antibody is used for characterization to insure lethal bioeffects in the animal bioassay are explicitly not the result of paralysis induced by neurotoxin. Characterization of the preparation prior to dilution is accomplished with gel electrophoresis, nucleic acid content assay, specific activity assay, and protein content assay for purity and consistency. Dilution accompanied by ultrafiltration establishes sterility. A region is targeted by the physician intending to treat a body region afflicted with the inflammatory process. A known selected bioactivity measured in LD 50 units is injected into the region. After 10 days, the region is inspected for the cardinal signs of inflammation. Booster doses may be given if necessary.

## Claims

1. A clostridial toxin which substantially lacks neurotoxic activity or a biologically active component thereof which substantially lacks neurotoxic activity, for use in therapy.

2. A toxin as claimed in claim 1 which is a botulinum toxin.

3. A botulinum toxin as claimed in claim 2 being type C2 botulinum toxin or a protein component I or II thereof.

4. A toxin as claimed in any one of claims 1 to 3 wherein said therapy is for the treatment of pain, irritation or inflammation or of diseases or disorders associated with pain, irritation or inflammation, or movement disorders.

5. A toxin as claimed in any preceding claim wherein said therapy is for the treatment of an essential headache disorder, photophobia, myofascial pain, regional muscle pain, dystonia, head or neck pain, back pain, trigeminal neuralgia or temporal mandibular joint syndrome.

6. A toxin as claimed in any preceding claim wherein said therapy is for the treatment of migraine or tension headaches.

7. A toxin as claimed in any preceding claim wherein said therapy is for the treatment of inflammation, pain, edema, redness or itching.

8. A toxin as claimed in any preceding claim wherein said therapy is for the treatment of allergic conjunctivitis, ocular inflammation, allergic blepharosconjunctivitis, asthma, eczema, hayfever, excessive salivation, tearing, otitis, anal fissures, cystitis, irritation, erythema or cholinergic urticaria.

9. A toxin as claimed in any one of claims 1-7 wherein said therapy is for the treatment of gastrointestinal ulceration, gastric, small or large intestinal inflammation, rheumatoid arthritis, vasculitis, blepharospasm, neurologic blepharospasm, hemifacial spasm, spasticity, cerebral palsy, multiple sclerosis, spasmodic torticollis, achalasia and bruxism.

10. A botulinum toxin as claimed in any one of claims 2 to 9, being substantially free of the C1-type botulinum toxin.

11. A toxin as claimed in any preceding claim in the form of a liquid or freeze dried composition.

12. A toxin as claimed in any preceding claim, being capable of providing a therapeutic effect without producing muscular weakness.

13. A toxin as claimed in any preceding claim, wherein said toxin has an activity of about 5 to 20 LD50 units as measured using the Swiss Webster mouse bioassay.

14. A toxin as claimed in any one of claims 1 to 12, wherein said toxin has an activity of about 20 to about 40 LD50 units as measured using the Swiss Webster mouse bioassay.

15. A toxin as claimed in any preceding claim wherein said toxin is purified from a bacterial strain which produces at least one active protein component of said toxin.

16. A toxin as claimed in any preceding claim wherein said toxin is prepared by biochemical separation to remove substantially all neurotoxin properties.

17. A toxin as claimed in any preceding claims, wherein said toxin is purified from a bacterial strain expressing at least one cloned gene encoding a protein component of said toxin.

18. A toxin as claimed in any preceding claim wherein the therapeutic effect of said toxin is mediated by inhibition of secretion of autocoids from mast cells, without antagonizing neuromuscular transmission.

19. A toxin as claimed in any preceding claim wherein said toxin is derived from Clostridium botulinum type C2.

20. A toxin as claimed in any preceding claim wherein said toxin is in a form suitable for administration by injection.

21. A toxin as claimed in any one of claims 1 to 19 wherein said toxin is in a form suitable for administration by topical application.

22. A toxin as claimed in any one of claims 1 to 12 or 15 to 21 wherein said toxin is in a form suitable for administration by injection in a dose of between approximately 20 and approximately 2000 LD50 units, as measured using the Swiss Webster mouse bioassay.

23. A toxin as claimed in any one of claims 1 to 3 or 10 to 22 wherein said therapy is for the treatment of post operative head and neck pain, post operative pain within the skin-muscle surgical flaps, temporal mandibular joint diseases, post operative bone pain, or post operative gastro-intestinal pain.

24. A pharmaceutical composition comprising a clostridial toxin as defined in any one of claims 1 to 23 and a pharmaceutically acceptable carrier or diluent.

25. Use of a clostridial toxin as defined in any one of claims 1 to 3 or 10 to 22 for the manufacture of a medicament for the treatment of pain, irritation or inflammation or of diseases and disorders which are associated with pain, inflammation and irritation, or movement disorders.

26. A pharmaceutical composition as claimed in claim 24, wherein said composition further comprises human serum albumin and adjuvant Clostridium-derived proteins.
